# EUROPÄISCHE PATENTSCHRIFT

(11) **EP 3 822 624 B1**
(45) Veröffentlichungstag und Bekanntmachung des Hinweises auf die Patenterteilung: **17.01.2024**
(21) Anmeldenummer: 20197188.4
(22) Anmeldetag: 21.09.2020
(51) Int. Cl.: G01N 27/22, G01N 33/00

(54) **KAPAZITIVES SENSORELEMENT ZUR ERFASSUNG MINDESTENS EINER EIGENSCHAFT EINES FLUIDEN MEDIUMS IN MINDESTENS EINEM MESSRAUM UND VERFAHREN ZUR HERSTELLUNG DES SENSORELEMENTS**
CAPACITIVE SENSOR ELEMENT FOR DETECTING AT LEAST ONE PROPERTY OF A LIQUID MEDIUM IN AT LEAST ONE MEASURING CHAMBER AND METHOD FOR MANUFACTURING THE SENSOR ELEMENT
ÉLÉMENT CAPTEUR CAPACITIF PERMETTANT DE DÉTERMINER AU MOINS UNE PROPRIÉTÉ D'UN MILIEU FLUIDE DANS AU MOINS UNE CHAMBRE DE MESURE ET PROCÉDÉ POUR FABRIQUER L'ÉLÉMENT DE CAPTEUR

(30) Priorität: 12.11.2019 DE 102019217465
(43) Veröffentlichungstag der Anmeldung: 19.05.2021
(73) Patentinhaber: Robert Bosch GmbH, 70442 Stuttgart (DE)
(72) Erfinder: Fuchs, Tino, 72076 Tuebingen (DE); Brenneis, Andreas, 71272 Renningen (DE)

(56) Entgegenhaltungen:
- EP-A1- 2 169 400
- EP-A1- 3 489 671
- HIROAKI YAMAZAKI ET AL: "High sensitivity MEMS capacitive hydrogen sensor with inverted T-shaped electrode and ring-shaped palladium alloy for fast response and low power consumption", JOURNAL OF MICROMECHANICS AND MICROENGINEERING, INSTITUTE OF PHYSICS PUBLISHING, BRISTOL, GB, Bd. 28, Nr. 9, 21. Mai 2018 (2018-05-21), Seite 94001, XP020330094, ISSN: 0960-1317, DOI: 10.1088/1361-6439/AAC21D [gefunden am 2018-05-21]
- BASELT D R ET AL: "Design and performance of a microcantilever-based hydrogen sensor", SENSORS AND ACTUATORS B: CHEMICAL, ELSEVIER BV, NL, Bd. 88, Nr. 2, 15. Januar 2003 (2003-01-15), Seiten 120-131, XP004399080, ISSN: 0925-4005, DOI: 10.1016/S0925-4005(02)00315-5

## Beschreibung

### Stand der Technik

Aus dem Stand der Technik ist eine Vielzahl von Sensorelementen und Verfahren zur Erfassung mindestens einer Eigenschaft eines fluiden Mediums in einem Messraum bekannt. Dabei kann es sich grundsätzlich um beliebige Eigenschaften eines gasförmigen oder flüssigen fluiden Mediums handeln, wobei eine oder mehrere Eigenschaften erfasst werden können. Die Erfindung wird im Folgenden, ohne Beschränkung weiterer Ausführungsformen und Anwendungen, insbesondere unter Bezugnahme auf Sensorelemente zur Erfassung eines Gases, insbesondere eines H₂-Anteils in einem Messgas, beschrieben.

Hiroaki et al., Baselt et al., EP 3 489 671 A1 und EP 2 169 400 A1 beschreiben jeweils Gassensoren mit einer festen Elektrode und einer Membran, an der eine bewegliche Elektrode angebracht ist. Diese Membran bildet über der festen Elektrode einen Hohlraum und überdeckt die feste Elektrode. Die Membran enthält ein gassensitives Material und wird deformiert, wenn das Material ein vorbestimmtes Gas absorbiert. Beide Elektroden werden bei Gasabsorption angenähert und eine Kapazität kann gemessen werden.

Sensorelemente der hier beschriebenen Art finden Anwendung in einer Vielzahl von Gebieten, beispielsweise in der Automobiltechnik, der Verfahrenstechnik, der Chemie und dem Maschinenbau, insbesondere zur Bestimmung von Gaskonzentrationen. So spielt beispielsweise die Bestimmung von Wasserstoffkonzentrationen, beispielsweise in einem Luft-Wasserstoff-Gemisch, bei der Anwendung von Wasserstoff-Brennstoffzellen-Systemen eine große Rolle. Hierbei sind auch sicherheitsrelevante Anwendungen zu nennen. Ein Luft-Wasserstoff-Gemisch wird etwa bei einem Wasserstoffanteil von 4 % zündungsfähig. Sensorelemente zur Erfassung von Wasserstoff können beispielsweise in Wasserstoff-Brennstoffzellenfahrzeugen zum Einsatz kommen, um beispielsweise aufgrund von Beschädigung oder Defekt austretenden Wasserstoff zu detektieren und, durch eine Kopplung an entsprechende Systeme, Warnsignale und/oder Schutzmaßnahmen auszulösen. Daher werden pro Brennstoffzellenfahrzeug mehrere Wasserstoffsensoren benötigt, die entweder im Abgasstrang angebracht werden (exhaust) oder unter atmosphärischen Bedingungen arbeiten (ambient).

Für derartige Wasserstoffsensoren kann man auf eine Vielzahl von Messprinzipien zurückgreifen. Dazu gehören u.a. folgende Messprinzipien: Wärmeleitung, katalytischer Pellistor, elektrochemische Zelle, halbleitendes Metalloxid, Chemiresistor, Feldeffekt Transistor.

Trotz der Vorteile der aus dem Stand der Technik bekannten Sensorelemente zur Erfassung mindestens einer Eigenschaft eines fluiden Mediums beinhalten diese noch Verbesserungspotenzial. Für die Verwendung in Automobiltechnik muss ein solcher Wasserstoffsensor bestimmte Anforderungen erfüllen. Die Verwendung der zuvor genannten Messprinzipien besitzen bezüglich dieser Anforderungen jeweils bestimmte Mängel bzw. Nachteile. So weisen derartige Sensorelemente überwiegend eine unzureichende Reaktionszeit, einen Messbereich oberhalb des minimalen Messbereichs und/oder eine Querempfindlichkeit gegenüber weiteren Komponenten wie Helium oder flüchtige organische Komponenten auf. Außerdem basieren diese teilweise auf einer teuren Aufbau- und Verbindungstechnik.

### Offenbarung der Erfindung

Im Rahmen der vorliegenden Erfindung wird daher ein Sensorelement zur Erfassung mindestens einer Eigenschaft eines fluiden Mediums in einem Messraum vorgeschlagen, welches die Nachteile bekannter Sensorelemente zur Erfassung mindestens einer Eigenschaft eines fluiden Mediums in einem Messraum zumindest weitgehend vermeidet, und das ausreichend Empfindlichkeit, Messbereich, Reaktionszeit und Selektivität bezüglich der Anforderungen in der Automobiltechnik bietet.

Unter einem Sensorelement wird im Rahmen der vorliegenden Erfindung grundsätzlich eine beliebige Vorrichtung verstanden, welche die mindestens eine Eigenschaft des fluiden Mediums erfassen kann und welche beispielsweise mindestens ein Messsignal entsprechend der erfassten Eigenschaft erzeugen kann, beispielsweise ein elektrisches Messsignal wie beispielsweise eine Spannung oder einen Strom. Bei der Eigenschaft kann es sich beispielsweise um eine physikalische und/oder eine chemische Eigenschaft handeln. Auch Kombinationen von Eigenschaften können erfassbar sein. Insbesondere kann das Sensorelement ausgestaltet sein zur Erfassung mindestens einer Eigenschaft eines Gases, insbesondere eines H₂-Anteils in einem Messgas.

Auch andere Eigenschaften und/oder Kombinationen von Eigenschaften können erfassbar sein.

Das Sensorelement kann insbesondere zum Einsatz in einem Wasserstoff-Brennstoffzellenfahrzeug eingerichtet sein. Bei dem Messraum kann es sich grundsätzlich um einen beliebigen, offenen oder geschlossenen, Raum handeln, in welchem das fluide Medium, insbesondere das Messgas, aufgenommen ist, und/oder welcher von dem fluiden Medium, insbesondere dem Messgas, durchströmt wird.

Das Sensorelement ist in Anspruch 1 offenbart.

Unter einer Membran kann im Rahmen der vorliegenden Erfindung eine dünne Struktur verstanden werden, die wie eine Haut oder Folie im Verhältnis zu ihrer Dicke eine große flächige Ausdehnung hat.

Die Messmembran kann parallel zu einer Oberseite des Substrats von der Isolationsschicht vorstehen. Damit ist zumindest das freie Ende der Messmembran relativ zu dem Substrat und der Isolationsschicht frei beweglich.

Die Isolationsschicht kann eine Dicke von 0,5 µm bis 15 µm und bevorzugt 1,0 µm bis 10,0 µm aufweisen. Alternativ oder zusätzlich kann die Membranschicht eine Dicke von 0,5 µm bis 7 µm und bevorzugt 1,0 µm bis 5,0 µm aufweisen. Alternativ oder zusätzlich kann die Deckschicht eine Dicke von 100 nm bis 2 µm und bevorzugt 200 nm bis 1,0 µm aufweisen. Damit lassen sich die genannten Schichten jeweils vergleichsweise dünne ausbilden, so dass das Sensorelement selbst vergleichsweise dünn herstellbar ist.

Die Deckschicht kann weiterhin Nickel aufweisen. Es ist insbesondere denkbar, die Palladium-Schicht mit Nickel zu vermischen, beispielsweise im Sputter-Prozess z.B. durch Cosputtern oder durch ein entsprechend legiertes Target. Dies erhöht die Widerstandsfähigkeit des Sensorelements gegen viele Ein- und Auslagerungszyklen von Wasserstoff. Grund hierfür ist, dass die Einlagerung von Wasserstoff in Palladium ab einer gewissen Wasserstoffkonzentration eine Phasenumwandlung erzeugt. Dies kann dazu führen, dass sich die dünne Deckschicht verformt mit der Tendenz einen isotropen Körper (Kugel bzw. Tröpfchen) zu bilden.

Das Substrat kann aus Silizium hergestellt sein. Alternativ oder zusätzlich kann die Isolationsschicht aus Siliziumoxid hergestellt sein. Alternativ oder zusätzlich kann die Membranschicht aus Silizium hergestellt sein. Damit lassen sich die genannten Bauteile aus Materialien herstellen, die einen identischen oder annährend identischen thermischen Ausdehnungskoeffizienten in dem Temperaturbereich aufweisen, in dem das Sensorelement betrieben wird.

Die Deckschicht kann auf einer dem Substrat abgewandten Seite der Membranschicht angeordnet sein.

Die Messmembran kann mit einem ersten Abschnitt einer Unterseite auf der Isolationsschicht angeordnet sein und ein zweiter Abschnitt der Unterseite kann frei beweglich relativ zu der Isolationsschicht sein, wobei der erste Abschnitt eine Fläche aufweist, die identisch groß oder unterschiedlich groß wie eine Fläche des zweiten Abschnitts ist. Damit ist die Messmembran mit einer vergleichsweisen Fläche an der Isolationsschicht befestigt. Die Kapazität und damit die Sensitivität des Sensorelements ist am größten, wenn der Abstand zwischen dem Substrat und der Membranschicht minimiert wird und die Fläche der dünnen Messmembran, die oberhalb des Substrats auf der verbleibenden Isolationsschicht liegt, minimiert wird.

In einem weiteren Aspekt wird ein Sensor vorgeschlagen, der ein Gehäuse und mindestens ein erstes Sensorelement gemäß den vorstehenden Ausführungen aufweist. Dabei ist das erstes Sensorelement zumindest teilweise in dem Gehäuse angeordnet. Das Gehäuse weist mindestens eine Öffnung auf, über die das erste Sensorelement mit dem fluiden Medium kontaktierbar ist.

Unter einem Gehäuse wird im Rahmen der vorliegenden Erfindung grundsätzlich ein beliebiges Bauteil oder eine Gruppe von Bauteilen verstanden, welche das Sensorelement ganz oder teilweise umschließen und/oder nach außen abschließen und dem Sensorelement eine mechanische Stabilität verleihen können. Insbesondere kann ein Gehäuse mindestens einen Innenraum umschließen. Beispielsweise kann das Gehäuse den Innenraum zumindest teilweise umschließen und ihn gegen seine Umgebung zumindest teilweise abgrenzen. Das Gehäuse kann insbesondere ganz oder teilweise aus mindestens einem der folgenden Materialien hergestellt sein: einem Halbleitermaterial, einem Kunststoff; einem Metall.

Der Sensor kann weiterhin ein zweites Sensorelement gemäß den vorstehenden Ausführungen umfassen, wobei das zweite Sensorelement zumindest teilweise in dem Gehäuse angeordnet ist, wobei das zweite Sensorelement gegenüber dem Messraum hermetisch abgetrennt ist oder die Deckschicht des zweiten Sensorelements mit einer Schicht bedeckt ist, die für das fluide Medium impermeabel ist. Es ist somit denkbar zur Kompensation von Temperatureffekten ein zweites Sensorelement vorzusehen, beispielsweise auf demselben Si-Chip zu integrieren, und das dem Wasserstoff nicht ausgesetzt wird. Dies dient dazu, dass mögliche Effekte aufgrund von beispielsweise unterschiedlichen thermischen Ausdehnungskoeffizienten aus der Messung herauskalibriert werden können. Das zweite Sensorelement kann sich beispielsweise entweder in einer geschlossenen Kammer befinden oder es ist denkbar, dass die Pd-Schicht des zweiten Sensorelements zusätzlich mit einem Material beschichtet ist, das verhindert, dass Wasserstoff in den Pd-Film diffundieren kann. Der monolithische Aufbau der beiden Sensorelemente ermöglicht, dass an beiden Sensorelementen vergleichbare Temperaturen herrschen. Damit können Temperatureffekte korrigiert werden.

Das Gehäuse kann ein Kappenwafer sein. Unter einem Kappenwafer ist im Rahmen der vorliegenden Erfindung ein beliebiger Wafer zu verstehen, der einen Innenraum zumindest teilweise begrenzt. Der Kappenwafer kann mindestens ein Material aufweisen ausgewählt aus der Gruppe bestehend aus: Silizium, Siliziumoxid, Siliziumnitrid und Siliziumcarbid.

In einem weiteren Aspekt wird ein Verfahren zum Herstellen eines Sensorelements nach Anspruch 9 vorgeschlagen.

Die Beweglichkeit der Messmembran kann durch teilweises Entfernen der Isolationsschicht realisiert werden Das Entfernen kann durch Ätzen erfolgen.

Im Rahmen der vorliegenden Erfindung werden die Ausdrücke "erster" und "zweiter" und dergleichen lediglich verwendet, um die jeweiligen Bauteile oder Merkmale begrifflich unterscheiden zu können, und sollen keine bestimmte Reihenfolge oder Gewichtung angeben.

Ein Grundgedanke der vorliegenden Erfindung ist ein alternatives Messprinzip, das darauf beruht, dass Wasserstoff sich mit einer besonders hohen Selektivität in Palladium einlagert und im Palladium Palladiumhydrid (H-Pd) bildet. Diese Einlagerung führt zu einer kompressiven Verspannung der Palladiumhydridschicht. Die kompressive Spannung und die daraus resultierende Verformung können statisch ausgelesen werden oder dynamisch durch eine Verschiebung der Resonanzfrequenz detektiert werden. So erhöht sich die Kapazität zwischen dem Substrat und der freigestellten Struktur in Form der Messmembran. Die elektrische Vermessung der Kapazität dient als Nachweis über die vorliegende Wasserstoffkonzentration.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
Figur 1 eine Querschnittsansicht eines Sensorelements in einem ersten Zustand,
Figur 2 eine Querschnittsansicht des Sensorelements in einem zweiten Zustand,
Figur 3 eine Draufsicht des Sensorelements,
Figur 4 eine Draufsicht eines Sensorelements gemäß einer Ausführungsform der vorliegenden Erfindung,
Figur 5 eine Querschnittsansicht eines Sensors und
Figuren 6A bis 6I Darstellungen von Verfahrensschritten der Herstellung des Sensorelements.

### Ausführungsformen der Erfindung

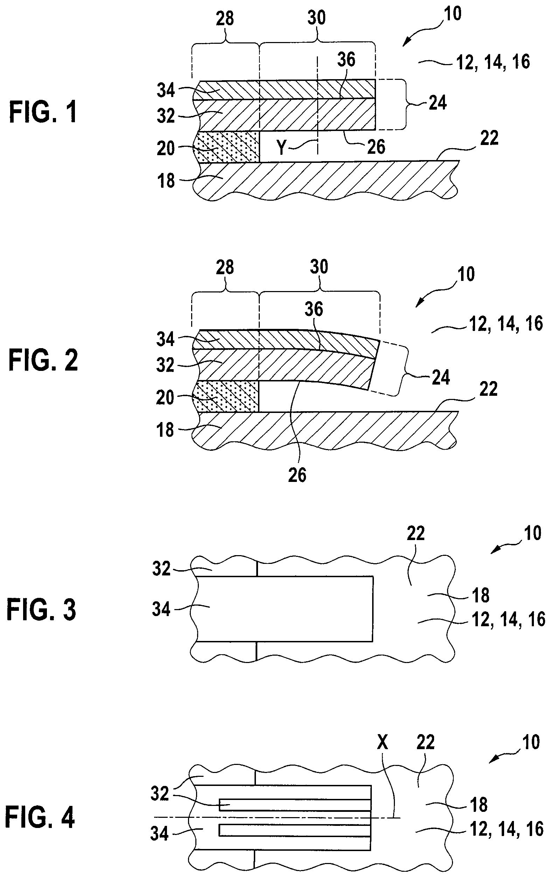

Figur 1 zeigt ein Sensorelement 10 gemäß einer ersten Ausführungsform der vorliegenden Erfindung in einer Querschnittsansicht. Das Sensorelement 10 kann insbesondere zum Einsatz in einem Wasserstoff-Brennstoffzellenfahrzeug eingerichtet sein. Auch andere Anwendungen sind jedoch möglich. Das Sensorelement 10 kann insbesondere ein oder mehrere in den Figuren nicht dargestellte, weitere Funktionselemente umfassen, wie beispielsweise Elektroden, Elektrodenzuleitungen und Kontakte, mehrere Schichten oder andere Elemente. Das Sensorelement 10 ist zur Erfassung mindestens einer Eigenschaft eines fluiden Mediums 12 in mindestens einem Messraum 14, insbesondere zur Erfassung eines H₂-Anteils in einem Messgas 16, ausgebildet, wie nachstehend ausführlicher beschrieben wird. Das Sensorelement 10 befindet sich in Figur 1 in einem ersten Zustand, der ohne Anwesenheit von Wasserstoff ist. Figur 2 zeigt eine Querschnittsansicht des Sensorelements 10 der ersten Ausführungsform in einem zweiten Zustand. In Figur 2 ist das Sensorelement 10 in einem zweiten Zustand gezeigt, der eine Anwesenheit von Wasserstoff im Messgas 16 anzeigt.

Das Sensorelement 10 umfasst ein Substrat 18. Das Substrat 18 ist zumindest teilweise aus einem elektrisch leitfähigen Material hergestellt, wie beispielsweise Silizium. Das Silizium ist bei diesem Beispiel hochdotiert, um eine ausreichende Leitfähigkeit vorzusehen, da das Substrat 18 später als Elektrode fungiert.

Das Sensorelement 10 umfasst weiterhin eine Isolationsschicht 20. Die Isolationsschicht 20 ist zumindest teilweise aus einem elektrisch isolierenden Material hergestellt, wie beispielsweise Siliziumoxid. Die Isolationsschicht 20 ist auf dem Substrat 18 angeordnet, insbesondere auf einer Oberseite 22 des Substrats 18. Dabei ist die Oberseite 22 des Substrats 18 nicht vollflächig von der Isolationssicht 20 bedeckt. Die Isolationsschicht 20 weist eine Dicke von 0,5 µm bis 15 µm und bevorzugt 1,0 µm bis 10,0 µm auf, wie beispielsweise 5 µm.

Das Sensorelement 10 umfasst weiterhin eine Messmembran 24. Die Messmembran 24 ist teilweise auf der Isolationsschicht 20 und zumindest teilweise relativ zu dem Substrat 20 beweglich angeordnet. Um die Beweglichkeit zu realisieren ist nur ein Teil einer Unterseite 26 der Messmembran 24 mit der Isolationsschicht verbunden. Insbesondere steht die Messmembran 24 parallel zu der Oberseite 22 des Substrats 18 von der Isolationsschicht 20 vor. Die Messmembran 24 ist insbesondere mit einem ersten Abschnitt 28 der Unterseite 26 auf der Isolationsschicht 20 angeordnet und ein zweiter Abschnitt 30 der Unterseite 26 ist relativ zu der Isolationsschicht 20 frei beweglich. Der erste Abschnitt 28 weist eine Fläche auf, die identisch groß wie eine Fläche des zweiten Abschnitts 30 sein kann. Alternativ kann der erste Abschnitt 28 eine Fläche aufweisen, deren Größe sich von einer Fläche des zweiten Abschnitts 30 unterscheidet. Mit anderen Worten kann die Fläche des ersten Abschnitts 28 größer als, kleiner als oder gleich groß wie eine Fläche des zweiten Abschnitts 30 sein. Beispielsweise weist der erste Abschnitt 28 eine deutlich kleinere Fläche als der zweite Abschnitt 30 auf, beispielsweise um mindestens Faktor 3 und bevorzugt mindestens Faktor 5 kleiner. Die Messmembran 24 weist eine Membranschicht 32 auf, die zumindest teilweise aus einem elektrisch leitfähigen Material hergestellt ist, wie beispielsweise Silizium. Die Membranschicht 32 weist eine Dicke von 0,5 µm bis 7 µm und bevorzugt 1,0 µm bis 5,0 µm auf, wie beispielsweise 2,0 µm. Die Messmembran 24 weist weiterhin eine Deckschicht 34 auf, die zumindest teilweise aus Palladium hergestellt ist. Die Deckschicht 34 ist in einem vorbestimmten Muster auf der Membranschicht 32 angeordnet. Die Deckschicht 34 ist auf einer dem Substrat 18 abgewandten Seite 36 der Membranschicht 32 angeordnet. Die Deckschicht 34 weist eine Dicke von 100 nm bis 2 µm und bevorzugt 200 nm bis 1,0 µm auf, wie beispielsweise 500 nm.

Figur 3 zeigt eine Draufsicht des Sensorelements 10. Bei einer nicht beanspruchten Ausführungsform ist das vorbestimmte Muster eine vollflächige Anordnung der Deckschicht 34 auf der Membranschicht 32. Optional kann die Deckschicht 34 weiterhin Nickel aufweisen. Wie in Figur 3 zu erkennen ist, können die Membranschicht 32 und die Deckschicht 34 unterschiedliche flächenmäßige Abmessungen aufweisen. Beispielsweise überragt die Membranschicht 32 die Deckschicht 34 seitlich im Bereich der Isolationsschicht 20.

Die Eigenschaft des fluiden Mediums und insbesondere ein Wasserstoffanteil in dem Messgas 16 ist basierend auf einer Kapazität zwischen dem Substrat 18 und der Messmembran 24 erfassbar. So lagert sich Wasserstoff mit einer besonders hohen Selektivität in Palladium ein und bildet im Palladium Palladiumhydrid (H-Pd). Diese Einlagerung führt zu einer kompressiven Verspannung der Palladiumhydridschicht. Entsprechend ist die Messmembran 24 in Figur 2 in Richtung zu dem Substrat 18 gekrümmt dargestellt. Entsprechend erhöht sich die Kapazität zwischen dem Substrat 18, das als eine Elektrode wirkt, und der freigestellten Struktur in Form der Messmembran 24, die als eine weitere Elektrode wirkt. Die elektrische Vermessung der Kapazität dient als Nachweis über die vorliegende Wasserstoffkonzentration. Dazu muss das Silizium-Substrat 18 ausreichend dotiert sein, um eine ausreichende Leitfähigkeit zur Vermessung der Kapazität bereitzustellen. Auch die dünne Si-Membranschicht 32 sollte durch entsprechende Dotierung ausreichend leitfähig sein, um die zweite Platte eines Kondensators zu bilden. Die Kapazität und damit die Sensitivität des Sensorelements 10 für Wasserstoff ist am größten, wenn der Abstand zwischen den beiden Siliziumschichten des Substrats 18 und der Membranschicht 32 minimiert wird und die Fläche des dünnen Pd/Si-Films als Messmembran 24, die oberhalb des Siliziums des Substrats 18 auf dem verbleibenden Siliziumoxid der Isolationsschicht 20 liegt, minimiert wird. Ferner sind für die Kontaktierung des Substrats 18 und der dünnen Deckschicht 34 Bondpads mit ggf. elektrischen Zuleitungen vorgesehen (nicht näher dargestellt).

Es ist auch denkbar, dass die Elektrode des Plattenkondensators auf der Substratseite durch eine zusätzliche Metallisierung unterhalb der freigestellten Messmembran realisiert wird.

Palladium besitzt eine besonders hohe Löslichkeit von Wasserstoff und ist zugleich selektiv auf die Einlagerung von Wasserstoff. Die Diffusionsgeschwindigkeit von Wasserstoff in Palladium ist maßgebend für die Reaktionsgeschwindigkeit eines Sensorelements 10, der auf der Diffusion von Wasserstoff in Palladium basiert. Mit der Diffusionskonstante *D* kann die zu erwartende Zeit Δ*t* berechnet werden, die benötigt wird damit Wasserstoff in eine gewisse Schichtdicke bzw. Schichttiefe *Δx* diffundiert. Dabei gilt **Δt** ≈ (**Δ*x***)²/***D***. Aus der Literatur ist der Diffusionskoeffizient als Funktion der Temperatur bekannt. Es zeigt sich zusätzlich, dass der Diffusionskoeffizient für dünne Pd-Schichten geringer ist. Dies führt dazu, dass die gleiche oder gar eine größere Strecke in einer kürzeren Zeit diffundiert wird, falls der abgeschiedene Pd-Film dicker ist. Die oben definierte dünne Dicke der Deckschicht 34 ergibt, dass die Ansprechzeitgeschwindigkeit (< 1 Sekunde) für die Anwendung als Wasserstoffsensor im Abgas oder in Umgebungsatmosphäre ausreichend schnell erfolgen kann.

Figur 4 zeigt eine Draufsicht eines Sensorelements 10 gemäß der Ausführungsform der vorliegenden Erfindung. Nachstehend werden lediglich die Unterschiede zu dem Beispiel aus Figur 3 beschrieben und gleiche oder vergleichbare Bauteile sind mit gleichen Bezugszeichen versehen. Bei der zweiten Ausführungsform ist das vorbestimmte Muster, in dem die Deckschicht 34 auf der Membranschicht 32 angeordnet ist, eine Streifenform. Die Streifen der Streifenform stehen dabei in einer Ebene parallel zu der Oberseite 22 des Substrats 20 von der Isolationsschicht 20 auf der Membranschicht 32 in einer ersten Richtung, angedeutet durch eine X-Achse vor. Die streifenförmige Ausbildung der Deckschicht 34 erlaubt, die Verformung der Messmembran 24 nur in die gewünschte Richtung hervorzurufen und einer unnötigen Verformung entlang einer orthogonalen, zweiten Richtung senkrecht zu der ersten Richtung in der Ebene parallel zu der Oberseite 22 des Substrats 18 zu vermeiden. Die Ausführung der Deckschicht in Streifen auf der freistehenden Struktur hat zur Folge, dass sich weniger kompressiver Stress in der durch eine Y-Achse (Figur 1) angedeuteten zweiten Richtung aufbaut.

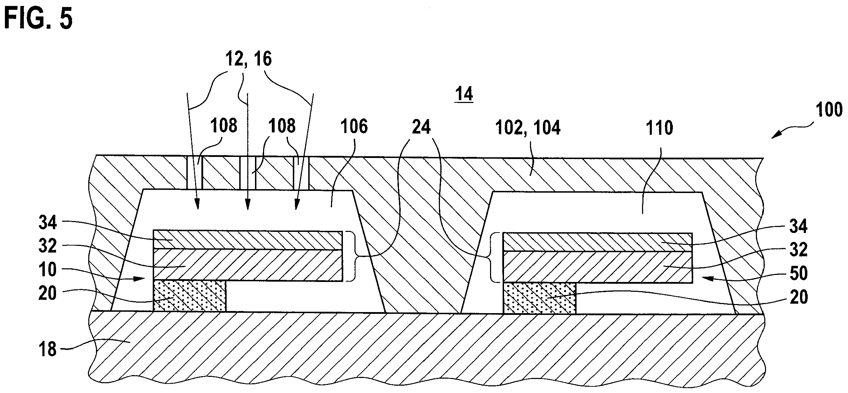

Figur 5 zeigt eine Querschnittsansicht eines Sensors 100. Der Sensor 100 weist ein Gehäuse 102 auf. Das Gehäuse ist bei dem gezeigten Ausführungsbeispiel ein sogenannten Kappenwafer 104. Das Gehäuse 102 definiert zumindest teilweise mindestens einen ersten Innenraum 106. Das Gehäuse 102 weist mindestens eine Öffnung 108 auf. Lediglich beispielhaft sind drei Öffnungen 108 in dem Gehäuse 102 vorgesehen. Es versteht sich, dass jedoch auch mehr oder weniger Öffnungen 108 vorgesehen sein können, wie beispielsweise genau eine Öffnung 108, zwei, vier, fünf oder noch mehr Öffnungen 108. Der Sensor 100 weist weiterhin mindestens ein erstes Sensorelement 10 auf. Das Sensorelement 10 kann ein Sensorelement 10 gemäß der ersten oder zweiten Ausführungsform sein. Das erstes Sensorelement 10 ist zumindest teilweise in dem Gehäuse 102 angeordnet. Wie gezeigt, ist das erste Sensorelement 10 in dem Innenraum 106 angeordnet, wobei das Substrat 18 und das Gehäuse 102 den Innenraum 106 begrenzen. Über die mindestens eine Öffnung 108 ist das erste Sensorelement 10 mit dem fluiden Medium 12 kontaktierbar. Beispielsweise kann Wasserstoff aus dem Messraum 14 über die mindestens eine Öffnung 108 in den Innenraum 106 gelangen und das erste Sensorelement 10 beaufschlagen.

Optional kann der Sensor 100 weiterhin ein zweites Sensorelement 50 aufweisen. Das zweite Sensorelement 50 kann einen identischen Aufbau wie das erste Sensorelement 10 aufweisen. Das Gehäuse 102 definiert einen zweiten Innenraum 110. Das zweite Sensorelement 50 ist zumindest teilweise in dem Gehäuse 102 angeordnet. So ist das zweite Sensorelement 50 in dem zweiten Innenraum 110 angeordnet. Das zweite Sensorelement 50 kann sich dabei das Substrat 18 mit dem ersten Sensorelement 10 teilen. Mit anderen Worten sind das erste Sensorelement 10 und das zweite Sensorelement 50 auf einem gemeinsamen Substrat 18 angeordnet. Das zweite Sensorelement 50 ist gegenüber dem Messraum 14 hermetisch abgetrennt. Alternativ kann die Deckschicht 34 des zweiten Sensorelements 50 mit einer Schicht (nicht näher gezeigt) bedeckt sein, die für das fluide Medium 12 und insbesondere Wasserstoff impermeabel ist. Durch das Anordnen in einer geschlossenen Kammer oder durch das Vorsehen der impermeablen Schicht wird verhindert, dass Wasserstoff in die Deckschicht 34 diffundieren kann. Das Vorsehen des zweiten Sensorelements 50, das dem fluiden Medium 12 und insbesondere Wasserstoff nicht ausgesetzt ist, dient dazu, dass mögliche Effekte aufgrund von beispielsweise unterschiedlichen thermischen Ausdehnungskoeffizienten aus der Messung herauskalibriert werden können. Der gezeigte monolithische Aufbau der beiden Sensorelemente 10, 50 ermöglicht, dass an beiden Sensorelementen 10, 50 vergleichbare Temperaturen herrschen. Damit können Temperatureffekte korrigiert werden.

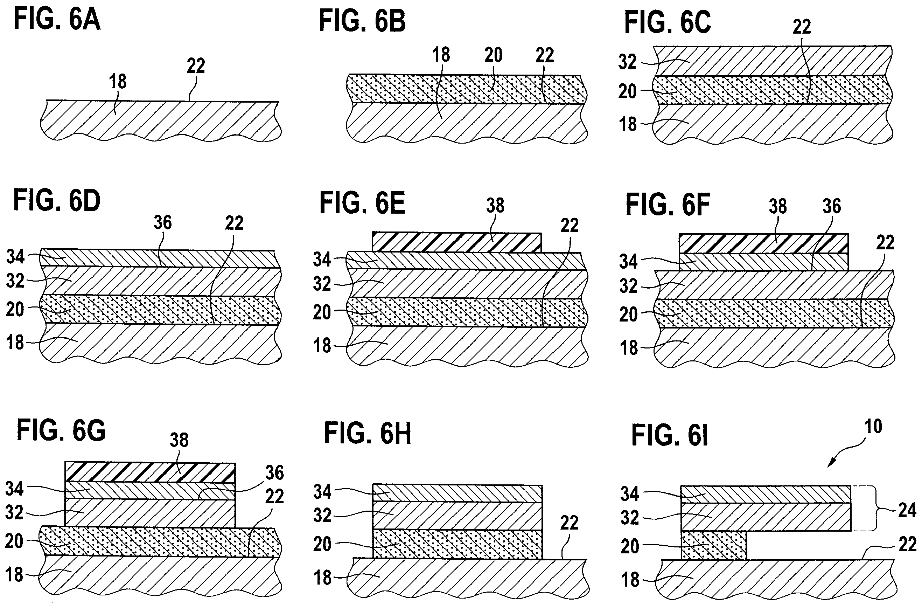

Figuren 6A bis 6I zeigen Darstellungen von Verfahrensschritten der Herstellung des Sensorelements 10 gemäß der Ausführungsform. Wie in Figur 6A gezeigt wird zunächst das Substrat 18 bereitgestellt. Wie in Figur 6B gezeigt, wird auf dem Substrat 18 die Isolationsschicht 20 angeordnet. Dies kann durch Erzeugen einer isolierenden Schicht, die z.B. aus Siliziumoxid hergestellt ist, entweder durch Aufbringen von Siliziumoxid z.B. durch Sputtern oder durch thermische Oxidation des Substrats 18 realisiert werden. Anschließend erfolgt ein teilweises Anordnen der Messmembran 24 auf der Isolationsschicht 22 derart, dass diese zumindest teilweise relativ zu dem Substrat 18 beweglich angeordnet ist, wie nachstehend ausführlicher beschrieben wird. Wir in Figur 6C gezeigt wird auf die Isolationsschicht 20 die Membranschicht 32 aufgebracht, wie insbesondere als Siliziumschicht wie z.B. Polysilizium. Es wird explizit betont, dass die in den Figuren 6A bis 6C gezeigten Verfahrensschritt auch durch einen sogenannten Silicon on Insulator SOl-Wafer erzeugt werden können. Wie in Figur 6D gezeigt wird auf der dem Substrat 18 abgewandten Seite 36 der Membranschicht 32 die Deckschicht 34 aufgebracht, wie beispielsweise durch Sputtern, Chemical Vapor Deposition (CVD), Aufdampfen durch Physical Vapor Deposition - PVD) oder Electroless plating (ELP), Electroplating deposition (EPD). Wie in Figur 6E gezeigt, wird erfolgt nachfolgend ein Aufbringen und Strukturieren einer Photolackmaske 38. Wie in Figur 6F gezeigt erfolgt anschließend ein Ätzen der freigestellten Deckschicht 34 z.B. durch lonenstrahlätzen, um das vorbestimmte Muster auszubilden. Wie in Figur 6G gezeigt erfolgt anschließend ein Trenchen der Membranschicht z.B. durch Reaktives lonen-Ätzen oder den Bosch-Prozess. Wie in Figur 6H gezeigt erfolgt anschließend ein Entfernen der Photolackmaske 38. Wie in Figur 6I gezeigt erfolgt abschließend ein Freistellen der Messmembran 24 wie beispielsweise durch Ätzen mit HF-Dampf. Dadurch steht die Messmembran 24 teilweise von der Isolationsschicht 20 parallel zu der Oberseite 22 des Substrats 18 vor und ist nur zu einem geringen Flächenanteil mit der Isolationsschicht 20 verbunden. Dadurch ist die Messmembran 24 relativ zu dem Substrat 18 beweglich.

Die erfindungsgemäße Ausbildung des Sensorelements 10 lässt sich sehr einfach durch optische Inspektion nachweisen. Die Wirkung kann unter dem Mikroskop quasi live beobachtet werden, wenn sich Wasserstoff einlagert. Durch eine Elementanalyse kann das Palladium als selektives Material an den entsprechenden Stellen im Sensorelement nachgewiesen werden. Elektroden, die zur kapazitiven Auslesung notwendig sind, können auch entweder optisch oder durch FIB-Schnitte im MEMS nachgewiesen werden.

## Patentansprüche

1. Sensorelement (10) zur Erfassung mindestens einer Eigenschaft eines fluiden Mediums (12) in mindestens einem Messraum (14), insbesondere zur Erfassung eines H₂-Anteils in einem Messgas (16), umfassend:
ein Substrat (18), das zumindest teilweise aus einem elektrisch leitfähigen Material hergestellt ist,
eine Isolationsschicht (20), die zumindest teilweise aus einem elektrisch isolierenden Material hergestellt ist und die auf dem Substrat (18) angeordnet ist, und
eine Messmembran (24), die teilweise auf der Isolationsschicht (20) und zumindest teilweise relativ zu dem Substrat (18) beweglich angeordnet ist, wobei die Messmembran (24) eine Membranschicht (32), die zumindest teilweise aus einem elektrisch leitfähigen Material hergestellt ist, und eine Deckschicht (34), die zumindest teilweise aus Palladium hergestellt ist und in einem vorbestimmten Muster auf der Membranschicht (32) angeordnet ist, aufweist, wobei die Eigenschaft des fluiden Mediums (12) basierend auf einer Kapazität zwischen dem Substrat (18) und der Messmembran (24) erfassbar ist, **dadurch gekennzeichnet, dass** das vorbestimmte Muster, in dem die Deckschicht (34) auf der Membranschicht (32) angeordnet ist, eine Streifenform ist,
mit mehreren in einer Ebene parallel zu einer Oberseite (22) des Substrats (18), ausgehend von der Isolationsschicht (20) auf der Membranschicht (32), in einer ersten Richtung vorstehenden Streifen.

2. Sensorelement (10) nach dem vorhergehenden Anspruch, wobei die Messmembran (24) parallel zu einer Oberseite (22) des Substrats (18) von der Isolationsschicht (20) vorsteht.

3. Sensorelement (10) nach einem der vorhergehenden Ansprüche, wobei die Isolationsschicht (20) eine Dicke von 0,5 µm bis 15 µm und bevorzugt 1,0 µm bis 10,0 µm aufweist und/oder wobei die Membranschicht (32) eine Dicke von 0,5 µm bis 7 µm und bevorzugt 1,0 µm bis 5,0 µm aufweist und/oder wobei die Deckschicht (34) eine Dicke von 100 nm bis 2 µm und bevorzugt 200 nm bis 1,0 µm aufweist.

4. Sensorelement (10) nach einem der vorhergehenden Ansprüche, wobei die Deckschicht (34) weiterhin Nickel aufweist.

5. Sensorelement (10) nach einem der vorhergehenden Ansprüche, wobei das Substrat (18) aus Silizium hergestellt ist und/oder wobei die Isolationsschicht (20) aus Siliziumoxid hergestellt ist und/oder wobei die Membranschicht (32) aus Silizium hergestellt ist.

6. Sensorelement (10) nach einem der vorhergehenden Ansprüche, wobei die Messmembran (24) mit einem ersten Abschnitt (28) einer Unterseite (26) auf der Isolationsschicht (20) angeordnet ist und ein zweiter Abschnitt (30) der Unterseite (26) frei beweglich relativ zu der Isolationsschicht (20) ist, wobei der erste Abschnitt (28) eine Fläche aufweist, die identisch groß oder unterschiedlich groß wie eine Fläche des zweiten Abschnitts (30) ist.

7. Sensor (100) mit einem Gehäuse (102), insbesondere einen Kappenwafer (104) und mindestens einem ersten Sensorelement (10) nach einem der vorhergehenden Ansprüche, wobei das erste Sensorelement (10) zumindest teilweise in dem Gehäuse (102) angeordnet ist, wobei das Gehäuse (102) mindestens eine Öffnung (108) aufweist, über die das erste Sensorelement (10) mit dem fluiden Medium kontaktierbar ist.

8. Sensor (100) nach dem vorhergehenden Anspruch, weiterhin umfassend ein zweites Sensorelement (50) nach einem der Ansprüche 1 bis 6, wobei das zweite Sensorelement (50) zumindest teilweise in dem Gehäuse (102) angeordnet ist, wobei das zweite Sensorelement (50) gegenüber dem Messraum (14) hermetisch abgetrennt ist oder die Deckschicht (34) des zweiten Sensorelements (50) mit einer Schicht bedeckt ist, die für das fluide Medium impermeabel ist.

9. Verfahren zum Herstellen eines Sensorelements (10) nach einem der Ansprüche 1 bis 6, umfassend die folgenden Schritte:
- Bereitstellen eines Substrats (18), das zumindest teilweise aus einem elektrisch leitfähigen Material hergestellt ist,
- Anordnen einer Isolationsschicht (20), die zumindest teilweise aus einem elektrisch isolierenden Material hergestellt ist, auf dem Substrat (18), und
- teilweises Anordnen einer Messmembran (24) auf der Isolationsschicht (20) und zumindest teilweise relativ zu dem Substrat (18) beweglich, insbesondere durch teilweises Entfernen der Isolationsschicht (20), wobei die Messmembran (24) eine Membranschicht (32), die zumindest teilweise aus einem elektrisch leitfähigen Material hergestellt ist, und eine Deckschicht (34), die zumindest teilweise aus Palladium hergestellt ist und in einem vorbestimmten Muster auf der Membranschicht (32) angeordnet ist, aufweist, wobei das vorbestimmte Muster, in dem die Deckschicht (34) auf der Membranschicht (32) angeordnet ist, eine Streifenform ist, mit mehreren in einer Ebene parallel zu einer Oberseite (22) des Substrats (18), ausgehend von der Isolationsschicht (20) auf der Membranschicht (32), in einer ersten Richtung vorstehenden Streifen .

## Claims

1. Sensor element (10) for detecting at least one property of a fluid medium (12) in at least one measuring chamber (14), in particular for detecting a proportion of H₂ in a measurement gas (16), comprising:
a substrate (18), which is at least partly produced from an electrically conductive material,
an insulation layer (20), which is at least partly produced from an electrically insulating material and which is arranged on the substrate (18), and
a measuring membrane (24), which is arranged partly on the insulation layer (20) and at least partly movably relative to the substrate (18), wherein the measuring membrane (24) has a membrane layer (32), which is at least partly produced from an electrically conductive material, and a cover layer (34), which is at least partly produced from palladium and is arranged in a predetermined pattern on the membrane layer (32), wherein the property of the fluid medium (12) is detectable on the basis of a capacitance between the substrate (18) and the measuring membrane (24), **characterized in that** the predetermined pattern in which the cover layer (34) is arranged on the membrane layer (32) is a strip shape with a plurality of strips projecting in a first direction in a plane parallel to a top side (22) of the substrate (18), proceeding from the insulation layer (20) on the membrane layer (32).

2. Sensor element (10) according to the preceding claim, wherein the measuring membrane (24) projects from the insulation layer (20) parallel to a top side (22) of the substrate (18).

3. Sensor element (10) according to either of the preceding claims, wherein the insulation layer (20) has a thickness of 0.5 µm to 15 µm and preferably 1.0 µm to 10.0 µm and/or wherein the membrane layer (32) has a thickness of 0.5 µm to 7 µm and preferably 1.0 µm to 5.0 µm and/or wherein the cover layer (34) has a thickness of 100 nm to to 2 µm and preferably 200 nm to 1.0 µm.

4. Sensor element (10) according to any of the preceding claims, wherein the cover layer (34) furthermore comprises nickel.

5. Sensor element (10) according to any of the preceding claims, wherein the substrate (18) is produced from silicon and/or wherein the insulation layer (20) is produced from silicon oxide and/or wherein the membrane layer (32) is produced from silicon.

6. Sensor element (10) according to any of the preceding claims, wherein the measuring membrane (24) is arranged with a first portion (28) of an underside (26) on the insulation layer (20) and a second portion (30) of the underside (26) is freely movable relative to the insulation layer (20), wherein the first portion (28) has an area with a magnitude identical to or different from that of an area of the second portion (30).

7. Sensor (100) having a housing (102), in particular a cap wafer (104), and at least a first sensor element (10) according to any of the preceding claims, wherein the first sensor element (10) is at least partly arranged in the housing (102), wherein the housing (102) has at least one opening (108) via which the first sensor element (10) is contactable with the fluid medium.

8. Sensor (100) according to the preceding claim, furthermore comprising a second sensor element (50) according to any of Claims 1 to 6, wherein the second sensor element (50) is at least partly arranged in the housing (102), wherein the second sensor element (50) is hermetically separated from the measuring chamber (14) or the cover layer (34) of the second sensor element (50) is covered with a layer that is impermeable to the fluid medium.

9. Method for producing a sensor element (10) according to any of Claims 1 to 6, comprising the following steps:
- providing a substrate (18), which is at least partly produced from an electrically conductive material,
- arranging an insulation layer (20), which is at least partly produced from an electrically insulating material, on the substrate (18), and
- partly arranging a measuring membrane (24) on the insulation layer (20) and at least partly movably relative to the substrate (18), in particular by partly removing the insulation layer (20), wherein the measuring membrane (24) has a membrane layer (32), which is at least partly produced from an electrically conductive material, and a cover layer (34), which is at least partly produced from palladium and is arranged in a predetermined pattern on the membrane layer (32), wherein the predetermined pattern in which the cover layer (34) is arranged on the membrane layer (32) is a strip shape with a plurality of strips projecting in a first direction in a plane parallel to a top side (22) of the substrate (18),
proceeding from the insulation layer (20) on the membrane layer (32).

## Revendications

1. Elément capteur (10) permettant de détecter au moins une propriété d'un milieu fluide (12) dans au moins une chambre de mesure (14), en particulier pour détecter une proportion de H₂ dans un gaz de mesure (16), comprenant :
un substrat (18) qui est fabriqué au moins partiellement à partir d'un matériau électriquement conducteur,
une couche d'isolation (20) qui est fabriquée au moins partiellement à partir d'un matériau électriquement isolant et qui est disposée sur le substrat (18), et
une membrane de mesure (24) qui est disposée partiellement sur la couche d'isolation (20) et de manière au moins partiellement mobile par rapport au substrat (18), dans lequel la membrane de mesure (24) présente une couche de membrane (32) qui est fabriquée au moins partiellement à partir d'un matériau électriquement conducteur, et une couche de couverture (34) qui est fabriquée au moins partiellement en palladium et qui est disposée selon un motif prédéterminé sur la couche de membrane (32), dans lequel la propriété du milieu fluide (12) peut être détectée sur la base d'une capacité entre le substrat (18) et la membrane de mesure (24), **caractérisé en ce que** le motif prédéterminé selon lequel la couche de couverture (34) est disposée sur la couche de membrane (32) est une forme de bande comprenant plusieurs bandes faisant saillie dans un plan parallèle à une face supérieure (22) du substrat (18), en partant de la couche d'isolation (20) sur la couche de membrane (32), dans une première direction.

2. Elément capteur (1) selon la revendication précédente, dans lequel la membrane de mesure (24) fait saillie à partir de la couche d'isolation (20) en parallèle à une face supérieure (22) du substrat (18).

3. Elément capteur (10) selon l'une quelconque des revendications précédentes, dans lequel la couche d'isolation (20) présente une épaisseur de 0,5 um à 15 um et de préférence de 1,0 µm à 10,0 um, et/ou dans lequel la couche de membrane (32) présente une épaisseur de 0,5 um à 7 um et de préférence de 1,0 µm à 5,0 um, et/ou dans lequel la couche de couverture (34) présente une épaisseur de 100 nm à 2 um et de préférence de 200 nm à 1,0 µm.

4. Elément capteur (10) selon l'une quelconque des revendications précédentes, dans lequel la couche de couverture (34) présente en outre du nickel.

5. Elément capteur (10) selon l'une quelconque des revendications précédentes, dans lequel le substrat (18) est fabriqué en silicium, et/ou dans lequel la couche d'isolation (20) est fabriquée en oxyde de silicium, et/ou dans lequel la couche de membrane (32) est fabriquée en silicium.

6. Elément capteur (10) selon l'une quelconque des revendications précédentes, dans lequel la membrane de mesure (24) est disposée avec une première partie (28) d'une face inférieure (26) sur la couche d'isolation (20), et une deuxième partie (30) de la face inférieure (26) est librement mobile par rapport à la couche d'isolation (20), dans lequel la première partie (28) présente une surface qui est de dimension identique ou différente par rapport à une surface de la deuxième partie (30).

7. Elément capteur (10) selon l'une quelconque des revendications précédentes, en particulier une plaquette de recouvrement (104) et au moins un premier élément capteur (10) selon l'une quelconque des revendications précédentes, dans lequel le premier élément capteur (10) est disposé au moins partiellement dans le boîtier (102), dans lequel le boîtier (102) présente au moins une ouverture (108) à travers laquelle le premier élément capteur (10) peut être mis en contact avec le milieu fluide.

8. Capteur (100) selon la revendication précédente, comprenant en outre un deuxième élément capteur (50) selon l'une quelconque des revendications 1 à 6, dans lequel le deuxième élément capteur (50) est disposé au moins partiellement dans le boîtier (102), dans lequel le deuxième élément capteur (50) est séparé hermétiquement par rapport à la chambre de mesure (14), ou la couche de couverture (34) du deuxième élément capteur (50) est recouverte d'une couche qui est imperméable pour le milieu fluide.

9. Procédé permettant de fabriquer un élément capteur (10) selon l'une quelconque des revendications 1 à 6, comprenant les étapes suivantes consistant à :
- fournir un substrat (18) qui est fabriqué au moins partiellement à partir d'un matériau électriquement conducteur,
- disposer une couche d'isolation (20), qui est fabriquée au moins partiellement à partir d'un matériau électriquement isolant, sur le substrat (18), et
- disposer partiellement une membrane de mesure (24) sur la couche d'isolation (20) et de manière au moins partiellement mobile par rapport au substrat (18), en particulier par un retrait partiel de la couche d'isolation (20), dans lequel la membrane de mesure (24) présente une couche de membrane (32) qui est fabriquée au moins partiellement à partir d'un matériau électriquement conducteur, et une couche de couverture (34) qui est fabriquée au moins partiellement en palladium et qui est disposée selon un motif prédéterminé sur la couche de membrane (32), dans lequel le motif prédéterminé selon lequel la couche de couverture (34) est disposée sur la couche de membrane (32) est une forme de bande comprenant plusieurs bandes faisant saillie dans un plan parallèle à une face supérieure (22) du substrat (18), en partant de la couche d'isolation (20) sur la couche de membrane (32),
dans une première direction.
